# EUROPEAN PATENT APPLICATION

(11) **EP 3 203 212 A1**
(43) Date of publication of application: **09.08.2017**
(21) Application number: 15846200.2
(22) Date of filing: 09.09.2015
(51) Int. Cl.: G01N 21/17, G01N 21/55, G01N 33/483, G01N 33/543

(54) **ANALYSIS DEVICE AND ANALYSIS METHOD**

(30) Priority: 30.09.2014 JP 2014200234
(71) Applicant: JVC Kenwood Corporation, Yokohama-shi, Kanagawa 221-0022 (JP)
(72) Inventor: ONO, Masayuki, Yokohama-shi, Kanagawa 221-0022 (JP); YAGYU, Shingo, Yokohama-shi, Kanagawa 221-0022 (JP); ITONAGA, Makoto, Yokohama-shi, Kanagawa 221-0022 (JP); HASEGAWA, Yuichi, Yokohama-shi, Kanagawa 221-0022 (JP); TSUJITA, Koji, Yokohama-shi, Kanagawa 221-0022 (JP)
(74) Representative: Schmidbauer, Andreas Konrad
(86) International application number: PCT/JP2015/075636
(87) International publication number: WO 2016/052118

(57) **Abstract**

An analysis device (1) includes an optical scanning unit (4) and a pulse waveform analysis unit (30). The optical scanning unit (4) optically scans a surface of a substrate (2) to which analytes (12) binding to particles (16) are fixed, and obtains a detection signal from the substrate (2). The pulse waveform analysis unit (30) includes an amplitude determination unit (32) configured to determine whether a peak value of a pulse waveform is within a predetermined range and a pulse width determination unit (33) configured to determine whether a pulse width of the pulse waveform is within a predetermined range, so as to analyze the pulse waveform of the detection signal.

## Description

### [TECHNICAL FIELD]

The present disclosure relates to an analysis device and an analysis method for analyzing biomaterials such as antibodies and antigens.

### [BACKGROUND ART]

Immunoassays are known to quantitatively analyze disease detection and therapeutic effects by detecting particular antigens or antibodies as biomarkers associated with diseases. One of the immunoassays is the enzyme-linked immunosorbent assay (ELISA) for detecting antigens or antibodies labeled by enzymes, and is widely used because of the advantage of low cost. The ELISA requires a long period of time, for example, from several hours to a day, to complete a series of multiple steps including pretreatment, antigen-antibody reaction, bond/free (B/F) separation, and enzyme reaction.

Another technology is disclosed in which antibodies that are fixed to an optical disc are allowed to bind to antigens in a specimen, the antigens are further bound to particles having antibodies and are then scanned with an optical head, so as to count the particles captured on the disc quickly (Patent Literature 1). Further, a technology is disclosed in which biosamples or particles are adsorbed to the surface of an optical disc on which a tracking structure is formed, so as to detect changes of signals by an optical pickup (Patent Literature 2).

### [CITATION LIST]

### [PATENT LITERATURE]

Patent Literature 1: Japanese Unexamined Patent Application Publication No. H05-005741
Patent Literature 2: Japanese Translation of PCT International Application Publication No. 2002-530786

### [SUMMARY OF INVENTION]

The analysis device using the optical disc causes noise components derived from fine scratches on the surface of the substrate of the optical disc when detecting biomarkers as analytes.

The analysis device using the optical disc further causes noise derived from an antigen-antibody reaction induced on the surface of the substrate of the optical disc. More particularly, residues, which are derived from the process of the fixation of antibodies to the surface of the substrate, blocking treatment with a solution such as skimmed milk and bovine serum albumin, or assaying with a saline buffer solution, remain on the optical disc after washing which causes noise components. The noise level derived from the residues is higher in one or more digits than the noise level derived from the fine scratches on the surface of the substrate. The residues thus lead to serious degradation in the accuracy of detecting biomarkers.

An object of an embodiment is to provide an analysis device and an analysis method with reduced influence of noise components, and improved accuracy in the detection of analytes with fine particles.

A first aspect of the embodiment provides an analysis device including: an optical scanning unit configured to optically scan a surface of a substrate to which analytes binding to particles are fixed, and obtain a detection signal from the substrate; and a pulse waveform analysis unit configured to analyze a pulse waveform of the detection signal obtained by the optical scanning unit, wherein the pulse waveform analysis unit includes: an amplitude determination unit configured to determine whether a peak value of the pulse waveform is within a predetermined range; and a pulse width determination unit configured to determine whether a pulse width of the pulse waveform is within a predetermined range.

A second aspect of the embodiment provides an analysis method including: a detection signal acquisition step of optically scanning a surface of a substrate to which analytes binding to particles are fixed, and obtaining a detection signal from the substrate; an amplitude determination step of determining whether a peak value of a pulse waveform of the detection signal is within a predetermined range; and a pulse width determination step of determining whether a pulse width of the pulse waveform is within a predetermined range.

The analysis device and the analysis method according to the embodiment can minimize the influence of noise components to improve the accuracy in the detection of analytes with fine particles.

### [BRIEF DESCRIPTION OF DRAWINGS]

[Fig. 1] Fig. 1 is a block diagram of an analysis device according to an embodiment.
[Fig. 2] Fig. 2 is a schematic view for describing an example of a method for capturing and sandwiching antigens as biomarkers between beads and a substrate by an antigen-antibody reaction to label and detect the antigens.
[Fig. 3] Fig. 3 is an example of data of a pulse waveform of a detection signal.
[Fig. 4] Fig. 4 is a flowchart for describing an analysis method according to the embodiment.
[Fig. 5] Fig. 5 is a schematic view showing a relationship between a pulse waveform and each parameter.
[Fig. 6] Fig. 6 is a view showing pulse waveforms for describing a method of setting each reference value.
[Fig. 7] Fig. 7 is a view showing a pulse waveform for describing a method of setting a reference value.
[Fig. 8] Fig. 8 is a correlation diagram showing a relationship between the count of beads and the amount of biomarkers.

### [MODES FOR CARRYING OUT THE INVENTION]

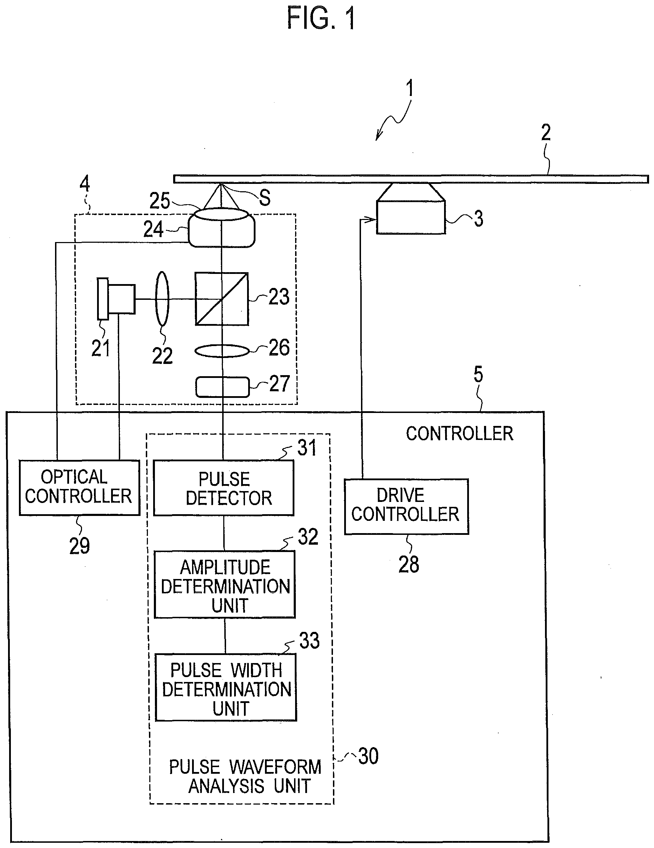

An analysis device according to the present embodiment will be described with reference to Fig. 1.

As shown in Fig. 1, the analysis device 1 includes a substrate 2, a drive unit 3 such as a motor that rotates the substrate 2, an optical scanning unit 4 that optically scans the surface of the substrate 2, and a controller 5 that controls the drive unit 3 and the optical scanning unit 4 and analyzes the pulse waveforms of detection signals detected by the optical scanning unit 4. The controller 5 may be a microprocessor or a microcomputer.

The substrate 2 is formed into a circular shape having substantially the same dimensions as optical discs such as compact discs (CDs), digital versatile discs (DVDs), and Blu-ray discs (BDs). The substrate 2 is formed of hydrophobic resin material, such as polycarbonate resin and cycloolefin polymer, used for common optical discs.

The substrate 2 has a track structure on the surface scanned by the optical scanning unit 4. The track structure includes grooves, lands, and pits, and has a spiral configuration extending from the inner side to the outer side. The substrate 2 may be provided with a thin film on the surface thereof or subjected to surface treatment with a silane coupling agent as necessary.

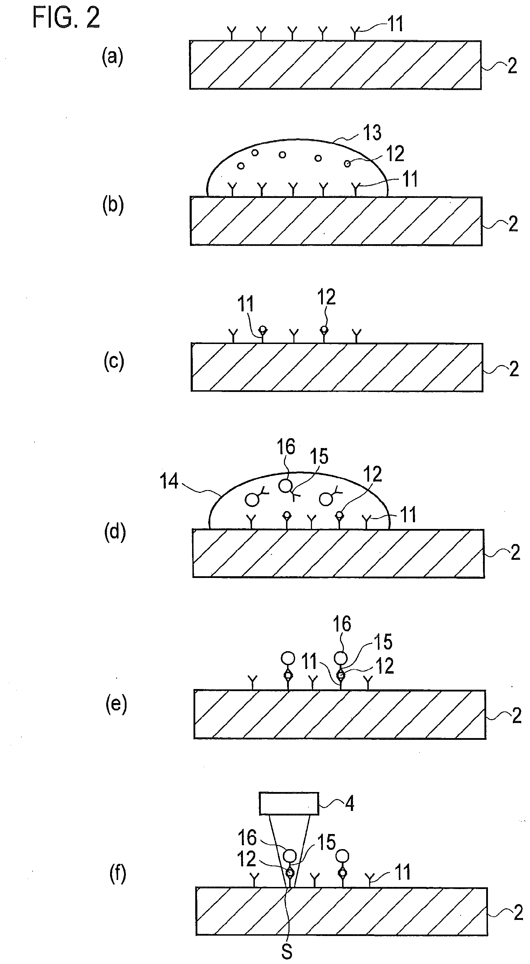

As shown in Fig. 2, antibodies 11 specifically binding to antigens 12, which are biomaterials as analytes, are fixed to the surface of the substrate 2. As described below, the antigens 12 are specifically bound to the antibodies 11 fixed to the substrate 2 and antibodies 15 fixed to the surface of beads 16 of fine particles. The antigens 12 that are captured and sandwiched between the beads 16 and the substrate 2 are labeled on the substrate 2. The antigens 12, specifically bound to the antibodies 11 and 15, are used as biomarkers serving as indicators of diseases.

As shown in (a) of Fig. 2, the antibodies 11 are preliminarily fixed to the surface of the substrate 2. The antibodies 11 are bound to the substrate 2 by hydrophobic binding or covalent binding. The antibodies 11 may be fixed to the surface of the substrate 2 via a substance such as avidin that has strong bonding force.

As shown in (b) of Fig. 2, a sample solution 13 including the antigens 12 is applied dropwise to the surface of the substrate 2. The antigens 12 move through the sample solution 13 by Brownian motion and come into contact with the antibodies 11 so as to be specifically bound to the antibodies 11 by an antigen-antibody reaction.

As shown in (c) of Fig. 2, the surface of the substrate 2 to which the sample solution 13 is applied dropwise is washed with pure water or the like, so as to remove the sample solution 13 including excessive antigens 12 not bound to the antibodies 11 from the surface of the substrate 2.

As shown in (d) of Fig. 2, a buffer solution 14 including the beads 16 is applied dropwise to the surface of the substrate 2. The buffer solution 14 may be applied while the sample solution 13 remains on the surface of the substrate 2. The antigens 12 as biomarkers are specifically bound to the antibodies 15 fixed to the beads 16 and the antibodies 11 fixed to the substrate 2, so as to be captured and sandwiched between the beads 16 and the substrate 2 and labeled on the substrate 2.

The beads 16 are formed of synthetic resin such as polystyrene including magnetic material such as ferrite, and have a substantially spherical shape. The diameter of the beads 16 is within a range of several tens of nanometers to about 200 nm. When the buffer solution 14 is applied dropwise, the beads 16 may be quickly collected to the surface of the substrate 2 such that a magnet is placed on the opposite side of the surface of the substrate 2 to promote the reaction between the beads 16 and the antigens 12. In addition, the time required to label the antigens 12 fixed to the substrate 2 can be reduced to approximately several minutes such that the antigens 12 and the beads 16 are simultaneously applied to the substrate 2.

The antibodies 11 and 15 are any biomaterials having specificity that specifically bind to the antigens 12. The antibodies 11 and 15 separately binding to different sites may be selected and combined.

For example, when membrane vesicles such as exosomes on which several types of antigens 12 are expressed are analytes, different types of the antibodies 11 and 15 are chosen so that a biosample including two types of antigens 12 can be analyzed. The antibodies 11 and 15 are, however, not limited to different types, and may be the same type because exosomes, which are different from typical antigens, include multiple antigens of the same kind of proteins on the surface thereof.

As shown in (e) of Fig. 2, the substrate 2, to which the buffer solution 14 is applied dropwise, is washed with pure water, for example, to remove the buffer solution 14 including excessive beads 16 not bound to the antigens 12 from the substrate 2.

As shown in (f) of Fig. 2, the substrate 2 is optically scanned by the optical scanning unit 4 to detect the beads 16, so as to analyze the antigens 12 (biomarkers) labeled with the beads 16. For example, the amount (concentration) of biomarkers can be weighed.

Returning to Fig. 1, the optical scanning unit 4 includes a laser oscillator 21, a collimator lens 22, a beam splitter 23, an actuator 24, an objective lens 25, a condensing lens 26, and a light detector 27. The optical scanning unit 4 is an optical pickup that optically scans the substrate 2.

The laser oscillator 21 is controlled by the controller 5 to emit laser light to the collimator lens 22. The laser oscillator 21 is a semiconductor laser oscillator that emits laser light having, for example, a wavelength of 405 nm, which is the same as that for the reproduction of BD, and an output of about 1 mW.

The collimator lens 22 collimates the laser light emitted from the laser oscillator 21. The beam splitter 23 reflects the laser light collimated by the collimator lens 22 toward the objective lens 25.

The objective lens 25 concentrates the laser light transmitted via the beam splitter 23 on the surface of the substrate 2, to which the antibodies 11 are fixed, due to the operation of the actuator 24 according to the control by the controller 5 to image spot S. The objective lens 25 has a numerical aperture of 0.85, for example. The laser light concentrated by the objective lens 25 is reflected from the substrate 2 and then reaches the beam splitter 23.

The incident laser light passing through the beam splitter 23 further reaches the light detector 27 via the condensing lens 26. The condensing lens 26 concentrates the laser light reflected from the substrate 2 on the light detector 27. The light detector 27 is, for example, a photodiode to output, to the controller 5, a detection signal corresponding to the volume of the laser light reflected from the substrate 2.

The controller 5 includes a drive controller 28 that controls the drive unit 3, an optical controller 29 that controls the laser oscillator 21 and the actuator 24, and a pulse waveform analysis unit 30 that analyzes a pulse waveform of the detection signal output from the light detector 27.

The drive unit 3 is controlled by the drive controller 28 to rotate the substrate 2 at a constant linear velocity (CLV).

The actuator 24 is controlled by the optical controller 29 to move the optical scanning unit 4 in a radial direction of the substrate 2, so as to spirally scan the surface of the rotating substrate 2. The optical controller 29 detects errors such as focus errors (FE) or tracking errors (TE) from the detection signal output from the light detector 27. The optical controller 29 controls the actuator 24 and other components to appropriately scan the surface of the substrate 2 depending on the errors detected.

The pulse waveform analysis unit 30 includes a pulse detector 31, an amplitude determination unit 32, and a pulse width determination unit 33. The amplitude determination unit 32 may be an amplitude filter.

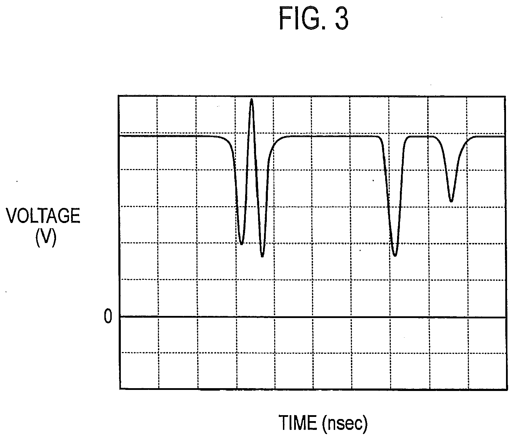

A method of analyzing, by the pulse waveform analysis unit 30 pulse waveforms of pulse signals output from the light detector 27, is described below with reference to Fig. 3 to Fig. 8.

An operator directs the drive controller 28 to control the drive unit 3 so as to rotate the substrate 2 at a predetermined constant linear velocity such as 4.92 m/s. The optical controller 29 controls the optical scanning unit 4 to concentrate laser light on the surface of the substrate 2 to image spot S. The substrate 2, to which the antigens 12 and the beads 16 are fixed by the antigen-antibody reaction, is rotated at the constant linear velocity by the drive unit 3, so as to be optically scanned by the optical scanning unit 4.

The optical scanning unit 4 directs the light detector 27 to detect the laser light emitted from the laser oscillator 21 and reflected from the surface of the substrate 2. The light detector 27 outputs a detection signal corresponding to the volume of the detected laser light to the pulse detector 31.

Fig. 3 illustrates a pulse waveform (of the detection signal) detected by the light detector 27. The transverse axis in Fig. 3 represents a time, and the vertical axis represents a voltage. The pulse waveform shown in Fig. 3 includes four downward peaks.

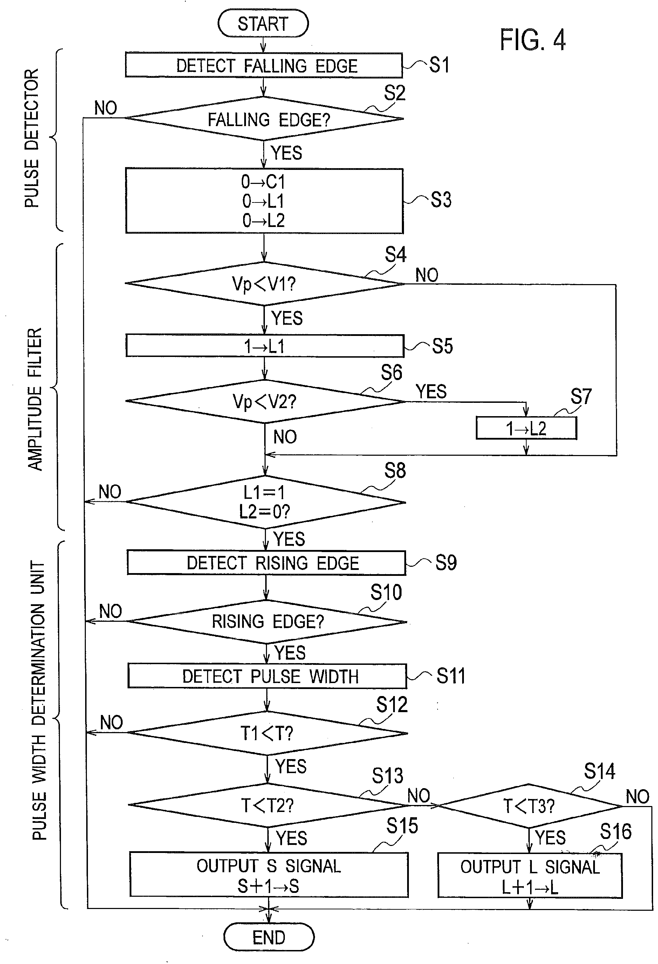

In step S1 shown in Fig. 4, the pulse detector 31 obtains the detection signal output from the light detector 27 to detect a falling edge of a pulse waveform of the obtained detection signal. The falling edge of the pulse waveform detected by the pulse detector 31 is a point where the pulse waveform falls below a predetermined threshold level (voltage) Vth.

The pulse detector 31 preliminarily holds a voltage as the threshold level Vth, corresponding to an average full width at half maximum (hereinafter, a full width at half maximum is abbreviated to "FWHM") of pulse waveforms of the beads 16 detected by the light detector 27.

In step S2, when the pulse detector 31 detects the falling edge of the pulse waveform (YES), the process proceeds to step S3. When the falling edge is not detected (NO), the pulse detector 31 ends the process.

In step S3, the pulse detector 31 obtains the time t1 at the point where the pulse waveform falls below the threshold level Vth and resets a counter flag C1, a flag L1 of a first reference level (voltage) V1, and a flag L2 of a second reference level (voltage) V2 (to "Low: 0"), and the process proceeds to step S4.

In step S4, the amplitude determination unit 32 detects a peak value (lowest voltage) Vp of the pulse waveform, and determines whether the detected peak value Vp is smaller than the predetermined first reference level V1.

When the amplitude determination unit 32 determines that the peak value Vp is smaller than the first reference level V1 (YES), the process proceeds to step S5. When the peak value Vp is larger than the first reference level V1 (NO), the process proceeds to step S8.

In step S5, the amplitude determination unit 32 sets the flag L1 to "High: 1", and the process proceeds to step S6.

In step S6, the amplitude determination unit 32 determines whether the peak value Vp of the pulse waveform is smaller than the predetermined second reference level V2. When the amplitude determination unit 32 determines that the peak value Vp is smaller than the first reference level V2 (YES), the process proceeds to step S7. When the peak value Vp is larger than the first reference level V2 (NO), the process proceeds to step S8.

In step S7, the amplitude determination unit 32 sets the flag L2 to "High: 1", and the process proceeds to step S8.

In step S8, when the amplitude determination unit 32 determines that the detection signal in which the flag L1 is "High: 1" and the flag L2 is "Low: 0" is an effective detection signal (YES), the process proceeds to step S9. The amplitude determination unit 32 ends the process when the detection signal is not effective (NO).

In step S9, the pulse width determination unit 33 detects a rising edge of the pulse waveform. The rising edge of the pulse waveform detected by the pulse width determination unit 33 is a point where the pulse waveform exceeds the threshold level Vth used in the falling edge detection.

In step S10, when the pulse width determination unit 33 detects the rising edge of the pulse waveform (YES), the process proceeds to step S11. The pulse width determination unit 33 ends the process when the rising edge is not detected (NO).

In step S11, the pulse width determination unit 33 obtains the time t2 at the point where the pulse waveform exceeds the threshold level Vth and obtains a pulse width T (substantially corresponding to FWHM) of the pulse waveform based on the obtained time t2 and the time t1 of the falling edge obtained in step S3, and the process proceeds to step S12.

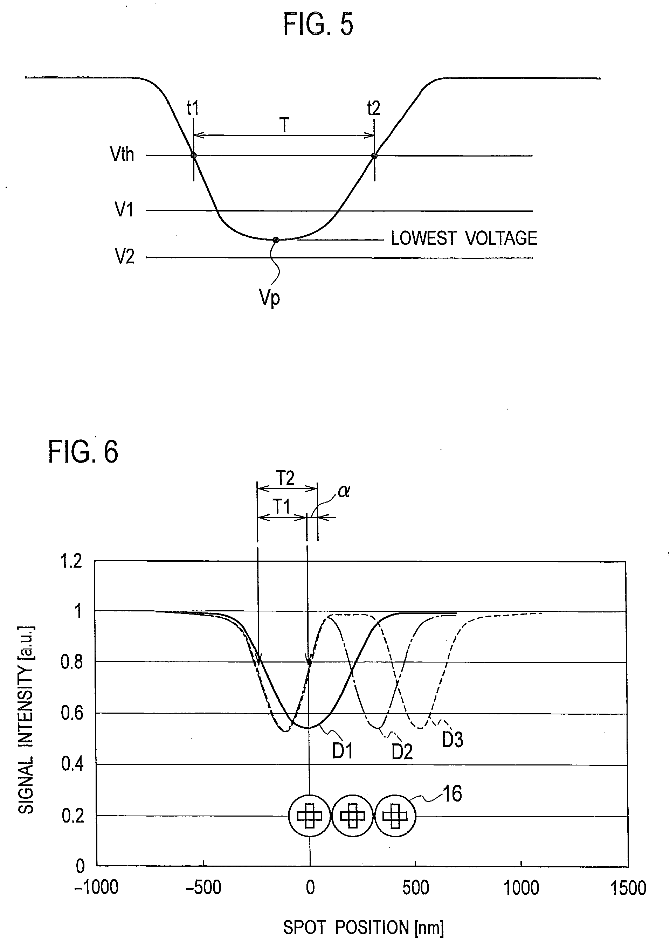

Fig. 5 illustrates a relationship between the peak value (the lowest voltage) Vp of the pulse waveform and the respective first and second reference levels V1 and V2, and a relationship between the pulse width T of the pulse waveform and each of the threshold level Vth, the time t1 of the falling edge, and the time t2 of the rising edge.

As shown in Fig. 5, the threshold level Vth, the first reference level V1, and the second reference level V2 have a relationship of Vth>V1>V2. The pulse waveform of the detection signal shown in Fig. 5 has the peak value Vp between the first reference level V1 and the second reference level V2. The detection signal shown in Fig. 5 is therefore determined as an effective detection signal in step S8.

The detection signal of the pulse waveform of which the peak value Vp is not present between the first reference level V1 and the second reference level V2 is determined as a noise component derived from an aggregation of proteins other than the analytes such as a blocking agent used for blocking non-specific binding.

For example, the left three of the four detection signals shown in Fig. 3 are determined as effective detection signals, each of which has the peak value Vp of the pulse waveform present between the first reference level V1 and the second reference level V2. The rightmost detection signal is determined as a noise component since the peak value Vp of the pulse waveform is not present between the first reference level V1 and the second reference level V2.

A method of setting the first reference level V1 and the second reference level V2 is described below. First, a proofing disc is prepared in which measurement beads are dispersed on a base plate of an optical disc, followed by drying, without fixation of antibodies to the substrate as described above with reference to Fig. 2, blocking treatment, or dropwise application of a saline buffer solution.

Next, the proofing disc is optically scanned by the optical scanning unit 4 in the analysis device 1 shown in Fig. 1, and pulse waves of detection signals are traced by an oscilloscope, for example, so that peak values (lowest voltages) of the pulse waves are measured. Thereafter, a variance for the measured peak values is calculated, and the range is set to 3σ, for example, so as to determine the first reference level V1 and the second reference level V2.

This setting method can easily and accurately determine the first reference level V1 and the second reference level V2 with the analysis device 1 used for the actual analysis based on the detection signals of beads actually used.

### [Analysis Result 1]

Detection signals of beads in which an average value of signal intensities (degrees of amplitude modulation) was 0.5 were subjected to filter treatment by the amplitude determination unit 32 under the conditions of Vth=0.75, V1=0. 6, and V2=0.3. As a result, approximately 2% of the detection signals were eliminated. The variance of the signal intensities of the detection signals of the beads seems to be 10% or less, that is, within the range of 0.45 to 0.55 in terms of the peak values (the lowest voltages). Namely, the detection signals eliminated by the amplitude determination unit 32 are recognized as being noise components. Accordingly, the accuracy of detecting biomarkers could be increased by approximately 2%.

### [Analysis Result 2]

An optical disc to which only noise components were fixed was prepared and assayed without dropwise application of a sample solution including antigens (biomarkers) as analytes. The optical disc was subjected to filter treatment by the amplitude determination unit 32 under the conditions of V1=0. 65 and V2=0.35. Accordingly, approximately 50% of the noise components could be eliminated, as compared with the case without use of the amplitude determination unit 32.

### [Analysis Result 3]

The same optical disc as used in the analysis result 2 was subjected to filter treatment by the amplitude determination unit 32 under the conditions of V1=0.57 and V2=0.45. Accordingly, approximately 80% of the noise components could be eliminated, as compared with the case without use of the amplitude determination unit 32. When the optical disc was assayed to which a sample solution including antigens (biomarkers) as analytes was applied dropwise and then subjected to filter treatment under the same conditions, part of the detection signals, which should have been counted, were eliminated as noise components.

According to the analysis results 1 to 3 described above, the filter treatment can be implemented appropriately in such a manner as to vary the conditions of filter treatment and set the first and second reference levels V1 and V2 as appropriate.

Returning to Fig. 4 in step S12, the pulse width determination unit 33 determines whether the obtained pulse width T is greater than the predetermined first reference width T1. When the pulse width determination unit 33 determines that the pulse width T is greater than the first reference width T1 (YES), the process proceeds to step S13. The pulse width determination unit 33 ends the process when the pulse width T is smaller than the first reference width T1 (NO).

In step S13, the pulse width determination unit 33 determines whether the pulse width T is smaller than the predetermined second reference width T2 (T2>T1). The pulse width determination unit 33 outputs the pulse signal S (in step S15) when the pulse width T is smaller than the second reference width T2 (YES), or sets the process proceeding to step S14 when the pulse width T is greater than the second reference width T2 (NO).

In step S14, the pulse width determination unit 33 determines whether the pulse width T is smaller than the predetermined third reference width T3 (T3>T2) . The pulse width determination unit 33 outputs the pulse signal L (in step S16) when the pulse width T is smaller than the third reference width T3 (YES), or ends the process when the pulse width T is greater than the third reference width T3 (NO).

Namely, the pulse width determination unit 33 detects the pulse waveform of which the peak value (the lowest voltage) is smaller than the first reference level and greater than the second reference level, and of which the pulse width is within a predetermined range, and outputs the pulse signal S or the pulse signal L depending on the pulse waveform detected.

The pulse signal S and the pulse signal L output from the pulse width determination unit 33 are counted by a counter (not shown), so as to analyze the amount (concentration) of biomarkers as analytes with a high accuracy. The counter may be included as a part of the controller 5, or may be included as an element secondary to the controller 5.

Further, the pulse signal S and the pulse signal L are separately counted and compared with each other, so as to confirm an aggregation state of beads. For example, the beads are aggregated as the proportion of the pulse signal S to the pulse signal L is greater, and the beads are dispersed as the proportion of the pulse signal S to the pulse signal L is smaller.

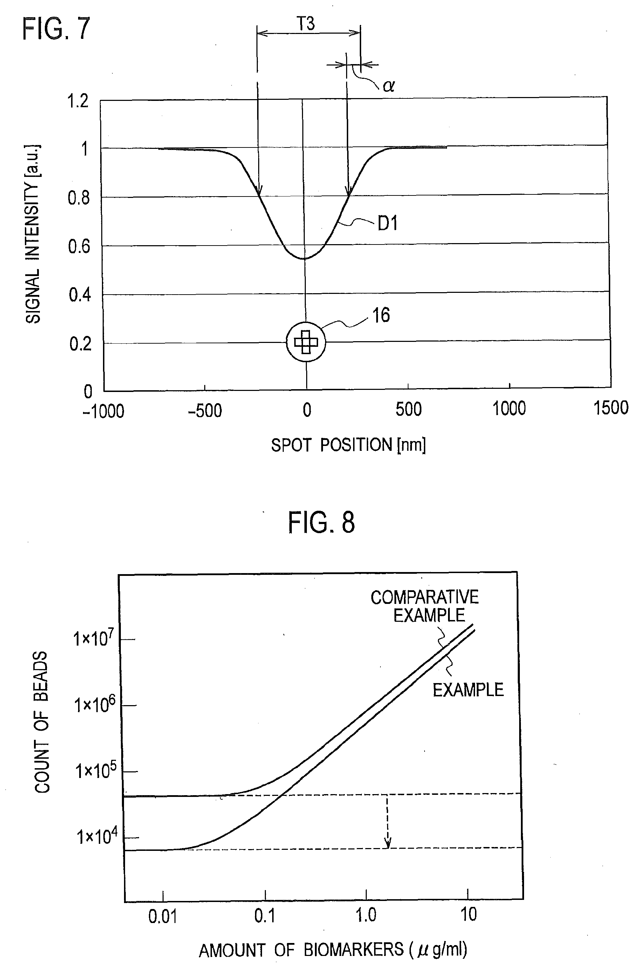

With reference to Fig. 6 and Fig. 7, a method of setting the first reference width T1, the second reference width T2, and the third reference width T3 is described below. The transverse axis in Fig. 6 and Fig. 7 represents a spot position, and the vertical axis represents signal intensity (the degree of amplitude modulation). Fig. 6 illustrates a pulse waveform D1 indicated by a solid line in which a single bead is located, a pulse waveform D2 indicated by a dash-dotted line in which two beads are aligned, and a pulse waveform D3 indicated by a broken line in which three beads are aligned. Fig. 7 illustrates only the pulse waveform D1 of Fig. 6 for illustration purposes.

As shown in Fig. 6, since the FWHM of the pulse waveform D2 is substantially the same as that of the pulse waveform D3, the FWHM of the pulse waveform in which a plurality of beads are aligned is determined as the first reference width T1. The second reference width T2 corresponds to the sum of the first reference width T1 and the value "α". The value "α" is determined in view of a jitter value of the analysis device, for example.

As illustrated in Fig. 7, the third reference width T3 corresponds to the sum of the FWHM of the pulse waveform D1 and the value "α". The value "α" is determined in view of a jitter value of the analysis device, for example.

According to the analysis device and the analysis method described above, the amplitude determination unit 32 analyzes the peak value (the lowest voltage) of the detection signal to eliminate a noise component, and the pulse width determination unit 33 analyzes the pulse width of the detection signal to further eliminate the remaining noise component that the amplitude determination unit 32 cannot eliminate. Accordingly, the analysis device and the analysis method can minimize the influence of the noise components more than the conventional devices and methods to improve the accuracy of detecting biomarkers.

The example by use of the analysis device 1 is described with reference to Fig. 8. The transverse axis in Fig. 8 represents the amount (concentration) of biomarkers, and the vertical axis represents the count of beads. Fig. 8 also illustrates the comparative example in which only the pulse width is analyzed without treatment by the amplitude determination unit.

The broken lines in Fig. 8 are background noise corresponding to noise components. The example and the comparative example show substantially the same inclination indicating the relationship between the count of beads and the amount of biomarkers. The background noise of the example is greatly below that of the comparative example. That is, the example can eliminate the noise components more than the comparative example. Accordingly, the example can improve the accuracy of detecting a slight amount of biomarkers.

It should be understood that the present embodiment is not intended to be limited to the configurations described above, and various modifications will be apparent to those skilled in the art without departing from the scope of the present invention. The controller 5 shown in Fig. 1 may include a circuit composed of at least part of the elements, each of which may optionally consist of either hardware or software.

### [INDUSTRIAL APPLICABILITY]

The present invention is applicable to a device and a method of analyzing biomaterials such as antibodies and antigens.

## Claims

1. An analysis device comprising:
an optical scanning unit configured to optically scan a surface of a substrate to which analytes binding to particles are fixed, and obtain a detection signal from the substrate; and
a pulse waveform analysis unit configured to analyze a pulse waveform of the detection signal obtained by the optical scanning unit, wherein
the pulse waveform analysis unit comprises:
an amplitude determination unit configured to determine whether a peak value of the pulse waveform is within a predetermined range; and
a pulse width determination unit configured to determine whether a pulse width of the pulse waveform is within a predetermined range.

2. The analysis device according to claim 1, wherein the pulse waveform analysis unit detects the pulse waveform of which the peak value is within the predetermined range and of which the pulse width is within the predetermined range.

3. An analysis method comprising:
a detection signal acquisition step of optically scanning a surface of a substrate to which analytes binding to particles are fixed, and obtaining a detection signal from the substrate;
an amplitude determination step of determining whether a peak value of a pulse waveform of the detection signal is within a predetermined range; and
a pulse width determination step of determining whether a pulse width of the pulse waveform is within a predetermined range.
